# EUROPEAN PATENT APPLICATION

(11) **EP 3 501 442 A1**
(43) Date of publication of application: **26.06.2019**
(21) Application number: 17210497.8
(22) Date of filing: 22.12.2017
(51) Int. Cl.: A61B 90/20, A61B 90/25, G02B 27/64, G02B 21/24, G02B 21/00, G06T 7/246

(54) **METHOD FOR OBSERVING AN OBJECT, NON-TRANSIENT COMPUTER READABLE STORAGE MEDIUM AND A MEDICAL OBSERVATION APPARATUS**

(71) Applicant: Leica Instruments (Singapore) Pte. Ltd., 608924 Singapore (SG)
(72) Inventor: Themelis, George, 88131 Lindau (DE)
(74) Representative: Grünecker Patent- und Rechtsanwälte PartG mbB

(57) **Abstract**

The invention relates to a method for observing an object (33) using a medical observation apparatus (1), such as a microscope (3), a non-transient computer readable storage medium (95) and a medical observation apparatus (1). Solutions of the art are expensive, bulky and prevents further usage of a microscope (3) is e.g. a robotic arm has a malfunction. The inventive method and medical observation apparatus (1) solves those problems by directing an optical assembly (7) to an object (33) located in a field of view (31), and by keeping the object (33) in focus when the optical assembly (7) is manually shifted, essentially perpendicularly to a viewing axis (17) of the optical assembly (7). The inventive apparatus (1) comprises an optical assembly (7) providing an optical viewing axis (17) and a lens adjustment assembly (29) which is configured to direct the optical assembly (7) to the object (33) in dependence on position data (65).

## Description

The invention relates to a method for observing an object using a medical observation apparatus, such as a microscope. The invention further relates to a non-transient computer readable storage medium and a medical observation apparatus, such as a microscope, for observing an object.

Methods of the prior art are known which utilize a surgical microscope with a robotic arm. The robotic arm moves and rotates an optics carrier in which an optical assembly is located. Movements are performed in such a way that the optical assembly is moved along the surface of a virtual sphere centered on the object under observation. Thus, the object may be observed from different angles. This method is known as point-lock. During the movement, the microscope, in particular the optics carrier with the optical assembly, is rotated such that the imaging axis always goes through the same point.

Such a robotic arm, however, has several drawbacks, for instance high costs, a bulky and heavy assembly as the robotic arm needs to be adapted to move the whole optics carrier, and the difficulty to retrofit an existing microscope with such a point-lock function. Furthermore, if the robotic arm stops due to a malfunction or fault, the microscope cannot be used to continue surgery until the robotic arm is fully operable again.

An object of the present invention is therefore to provide a method and a medical observation apparatus which are less costly, easier to use and more reliable.

The method mentioned in the beginning solves the above problems in that an optical assembly is directed to an object located in the field of view, and in that the object is kept automatically in the field of view when the optical assembly is manually moved essentially perpendicularly to a viewing axis of the optical assembly.

The non-transitory computer readable storage medium mentioned in the beginning solves the above problems by comprising a program for executing the method according to the invention.

The medical observation apparatus mentioned in the beginning solves the above problems in that it comprises an optical assembly providing an optical viewing axis and a field of view, and a housing for supporting the optical assembly, wherein the optical assembly is adapted to be moved essentially perpendicularly to the optical viewing axis from one position to another position and wherein a lens adjustment assembly is configured to be automatically directed to the object in dependence on position data representative of the position of the manually moved optical assembly.

The inventive method and medical observation apparatus have the advantage that they are easier and more intuitively to a surgeon. Furthermore they are more reliable and reduce the risk of interruptions of a surgery. Additionally, they require less space and may be retrofitted to already existing medical observation apparatusses.

The inventive method and apparatus is directed to a manual or driveless movement and/or moveability of the optical assembly. The situation when the medical observation apparatus, in particular its optical assembly, is moved manually differs thoroughly from the situation encountered when using a robotic arm. In the latter, the system has always knowledge about the current and subsequent position of the robotic arm from the known trajectory of the robotic arm. The present invention, to the contrary, aims to provide a medical observation device and method where such a predetermined trajectory cannot be used. During a manual movement of the optical assembly the angular orientation of the optical assembly is permanently readjusted.

The inventive method and the inventive medical observation apparatus may be improved by specific embodiments which will be described in the following. Technical features of individual embodiments may be arbitrarily combined with each other or may be omitted if the technical effect obtained by the omitted technical feature is not relevant to the present invention.

For example, the optical assembly, in particular a viewing axis of the optical assembly, may be continuously directed to, point at or be adjusted to one specific, preferably predetermined location in the field of view. In particular, the location may be the center of the object. Preferably, the location is always kept in focus and at the same location within the field of view.

In one embodiment of the inventive method the method comprises the steps of receiving position data from a position sensor and readjusting the optical assembly may be based on the position data from the position sensor.

The position sensor detects this repositioning and readjusts in particular at least one of the angular orientation and the nominal focal length of the optical assembly. The readjustment may preferably be based on the determined position data.

A corresponding embodiment of the inventive medical observation apparatus may comprise a position sensor for generating position data representing the position and/or angular orientation of the optical assembly is provided.

The position sensor, or briefly the sensor may have a position data interface for providing the position data.

Such a position sensor may comprise at least one of a gyroscope, accelerometer, tilt sensor, leveling sensor, incremental positioning encoder, absolute position encoder and linear distance sensor. The position sensor or sensors may be adapted to provide position data which unambiguously determine the position and/or angular orientation of the optical assembly.

The optical assembly may be mounted to a housing. The housing may be supported shiftable by a frame of the medical observation device. In particular, the housing may be guided for only translational movements, e.g. not be tiltable. This restriction facilitates the manual handling during manual movement of the optical assembly and shows a more accurate and repeatable positioning by hand.

Angular readjustment of the optical assembly is performed dependent on the position data, wherein, if an increase on decreased of the working distance (the distance between the optical assembly and the object under study) is detected, the inventive method and the inventive medical observation apparatus may be adapted to vary an effective focal length of the optical assembly in order to adapt to the changed working distance.

In a further embodiment, the method may comprise the steps of recognizing at least one pattern of the object in image data received from a camera and retrieving position data based on a variation of the at least one pattern over time in a time series of image data, wherein the position data are applied for readjusting the optical assembly to keep the object in focus.

In such a configuration, the position data may preferably be retrieved from images detected by the microscope. For this, the position sensor of the inventive medical observation apparatus may comprise a pattern recognition module for identifying at least one structure in the input image data.

Thus, the input image data, in particular predetermined points of the input image data or certain structures of the object are utilized, more specifically tracked for retrieving the position data. Here, known methods of triangulation may be applied. This method allows a real-time feedback to the lens adjustment assembly.

In a different embodiment of the inventive method, a marker may be applied to the object under study prior to or during the observation. The marker may comprise a pattern to be recognized by the pattern recognition module, wherein this pattern may preferably be stored as a predetermined pattern in the apparatus for comparison.

The inventive method may take into consideration that three-dimensional, i.e. non-flat objects yield different two-dimensional projections, i.e. appearances in the image data for different angles.

The inventive medical observation apparatus may provide a sufficiently high frame rate, such that a change of the two dimensional shape of the pattern may be followed. That is to say that a movement of a pattern needs to be correlated, i.e. retrievable between respectively from two subsequent housings. If the pattern is moved too far between two subsequent housings, the inventive method may not be able to determine the direction and the moment of the readjustment of the optical assembly.

The inventive method which may comprise the step of recognizing at least one pattern of the object may be further improved in that recognizing the at least one pattern comprises stereoscopic imaging of the object.

The according embodiment of the medical observation apparatus thus may comprise a pattern recognition module with a stereoscopic imaging module. The stereoscopic imaging module may comprise multiple cameras which further increase the performance of the inventive method and inventive medical observation apparatus.

The inventive method may be further improved in that it further comprises the steps of reading out assignment data from an assignment table, correlating the position data with the correction data and readjusting the optical assembly based on correlated correction data.

An embodiment of the inventive medical observation apparatus may comprise a storage module for storing assignment data which correlate position data with correction data, wherein the position of the optical assembly is adjusted based on the correction data.

The correction data may be stored in an assignment or look-up table.

The correction data may be understood as data that represents a necessary readjustment, i.e. an angular readjustment or a distance readjustment of the optical assembly in order to keep the object in focus, respectively to maintain the optical assembly directed to the object.

Consequently, such a readjustment may ensure, that the optical viewing axis of the optical assembly is tilted, such that it permanently projects through the predetermined point of the object under study.

In order to advantageously read out and process the correction data from the assignment table, a further embodiment of the inventive medical observation apparatus may be characterized in that the adjustment assembly comprises a controller having an input interface for receiving position data of the optical assembly, further having an output interface for providing correction data to a movable readjustment assembly for readjusting the optical assembly to keep the object in focus.

The controller may effectively combine different functionalities for performing the inventive method as for instance to read the correction data from the assignment table and to correlate the position data to the corresponding set of correction data read out from the assignment table as well as to control and to operate the lens adjustment assembly.

In yet a further embodiment of the inventive method, the method further comprises computing the correction data from the position data and readjusting the optical assembly on the calculated correction data.

The according embodiment of the inventive medical observation apparatus comprises a calculation module for computing correction data based on the position data, wherein the position of the optical assembly is adjusted based on the correction data.

The correction data is therefore not predetermined but is recalculated each time position data are provided to the controller, in particular to the calculation module.

In this embodiment is therefore not necessary to interpolate correction data read from the assignment table; quite the contrary is the case, the exact value of the correction data is calculated each time position data are provided.

In the following, the invention will be described using exemplary embodiments which are shown in the accompanied figures.

The embodiments that will be shown merely represent exemplary embodiments of the present invention. The given technical features may be arbitrarily combined, wherein different technical features may be omitted as well, as long as the technical effect obtained with the omitted technical feature is not relevant to the present invention. The same technical features or technical features having the same technical effect will be denoted with the same reference numeral. A repetitive description of already described technical features will be omitted. The described embodiments are to be understood as not limiting the scope of protection, which is defined by the claims.

In the figures
- Fig. 1: shows a schematic drawing of the inventive medical observation apparatus and its working principle; and
- Fig. 2: shows a simplified schematical representation of the inventive medical observation apparatus.

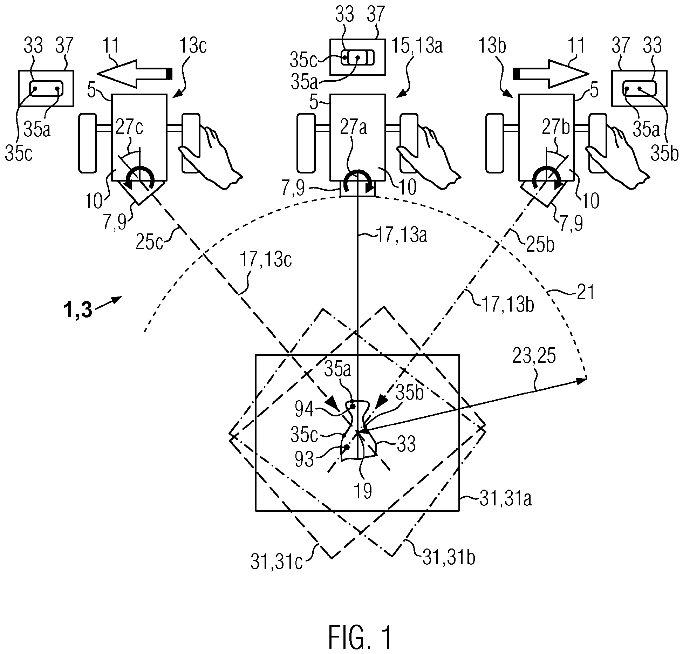

Fig. 1 illustrates the working principle of the inventive medical observation apparatus 1, which is embodied as a microscope 3.

The figure illustrates a manually moveable housing 5 which supports an optical assembly 7 which may consist of or comprise a lens system or objective 9, including devices for beam detection such as mirrors or prisms.

Fig. 1 further shows two possible movements 11 of the housing 5 and thereby also the optical assembly 7.

The optical assembly 7 (received within the housing 5) is shown in a first position 13a, a second position 13b and a third position 13c.

If the first position 13a represents the initial position 15, each of the movements 11 are oriented essentially perpendicularly to a viewing axis 17, wherein according to the position 13a-13c of the optical assembly 7, also the corresponding viewing axis 17 is in the first 13a, the second 13b or the third position 13c.

As can be seen, the housing 5 is shiftable with respect to a frame (not shown) of the medical observation apparatus 1. In particular, the housing 5 may be guided, e.g. by a parallelogram device (not shown), to be moved only translationally. Preferably, the housing itself is not tiltable, only the optical assembly 7 may be tiltable with respect to the housing 5, and be preferably not shiftable relative to the housing 5 perpendicular to the viewing axis 17.

The three viewing axes 17 cross each other in a center point 19, wherein this center point 19 defines a virtual sphere 21. A radius 23 of said virtual sphere 21 corresponds to a working distance 25 of the optical assembly 7 in the first position 13a.

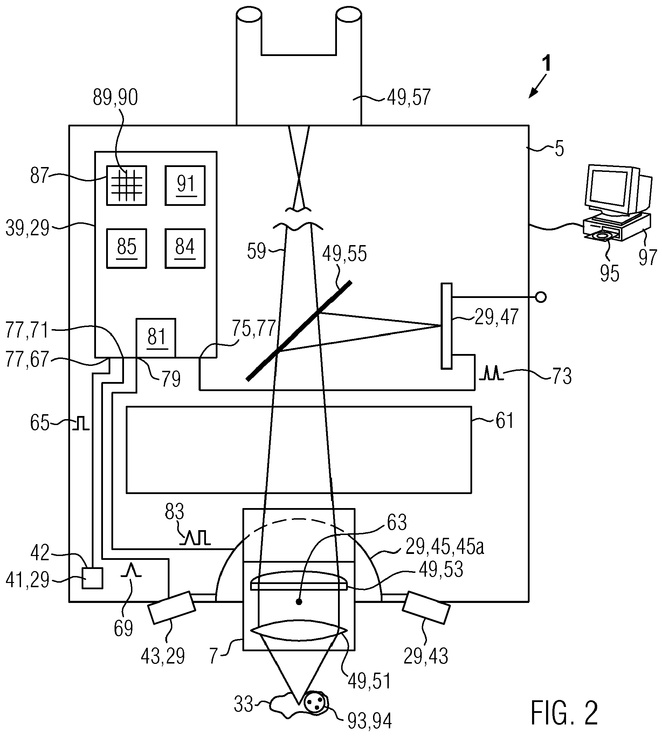

In the second 13b and third position 13c, a second 25b and third working distance 25c is set, respectively. Both working distances 25b and 25c are larger than the working distance 25. A description how a changed working distance 25 will be taken care of by the inventive medical observation apparatus 1 will be given in Fig. 2.

In order to achieve that the viewing axes 17 are directed to the center point 19 for each position 13a-13c, the optical assembly 7 is tilted.

In the first position 13a of the optical assembly 7, a first tilt angle 27a corresponds to zero degree, wherein in the second 13b and third position 13c a second 27b and a third tilt angle 27c are measured, respectively.

The tilt of the optical assembly 7 is performed by a lens adjustment assembly 29 which will be described in Fig. 2.

The optical assembly 7 defines a field of view 31 which is schematically shown in Fig. 1. In each position 13a-13c a first 31 a, a second 31 b and a third field of view 31 c are defined, respectively. Each field of view 31 extends into the drawing plane but is shown in a view as seen along the corresponding viewing axis 17.

It can be seen that the field of view 31 rotates around the center point 19, if an object 33 located in the field of view 31 is regarded from different angles. The optical assembly 7 is automatically tilted at each of the position 13a to 13c to maintain the essentially same field of view 31, in particular to be directed to always the same location in the field of view 31. Preferably, this location is at the center of the field of view 31. For effecting the tilting, a drive system (not shown) may be provided. The drive system may comprise a separate drive, such as an electric motor, for each rotational axis, about which the optical assembly 7 may be tilted.

This is described in more detail by a first reference point 35a, a second reference point 35b and a third reference point 35c which are exemplarily drawn.

An image 37 obtained for the three different positions 13a-13c is schematically shown, wherein in the first position 13a only the first 35a and third reference point 35c are shown, the Image 37 in the second position 13b shows the first 35a and the second reference point 35b, whereas in the third position 13c, the image 37 does not show the second reference point 35b.

It is to be noted that the reference points 35a-35c do not correspond to points of a structure used for pattern recognition. They are solely shown for explanation of the different perspective.

In Fig. 2 a simplified schematical representation of the inventive medical observation apparatus 1 is shown in more detail.

The housing 5 comprises the before mentioned lens adjustment assembly 29, which is composed of several components. Those components comprise (in the embodiment shown) a controller 39, a position sensor 41, two stereoscopic cameras 43, a rotational stage 45 and an image sensor 47.

An optical system 49 comprises one or more lenses 51 (only one is shown in Fig. 2), a tunable lens 53, a beamsplitter 55, the image sensor 47 and optical observation means 57.

An optical path 59 is drawn from the object 33 through the lens 51, through the tunable lens 53 and through a beam path correction assembly 61. The beam path correction assembly 61 assures that the optical path 59 through the beamsplitter 55 is not changed if the optical assembly 7 is rotated by the rotational stage 45. In the upper part of the figure, the optical path 59 is drawn discontinuously for the sake of the size of Fig. 2.

It is to be noted that the rotational stage 45 solely represents one possibility to tilt the viewing axis 17. Different means which are configured to tilt the viewing axis 17 are conceivable as well.

The optical assembly 7 is rotated around a center of rotation 63, wherein the arrangement of the lens 51 and the tunable lens 53 with respect to the center of rotation 63 may be different in a different embodiment of the medical observation apparatus 1.

The position sensor 41 provides position data 65 via a position data interface 42, which data 65 is represented by a rectangular shaped electric signal and which is provided to a position data input port 67 of the controller 39.

The stereoscopic cameras 43 deliver stereoscopic image data 69 represented by a triangular shaped electric signal, which are provided to the controller 39 via a stereoscopic image data input port 71.

The image sensor 47 generates image data 73 represented by an electric signal with two spikes, wherein the image data 73 may be input to the controller 39 via a image data input port 75.

The position data input port 67, the stereoscopic image data input port 71 and the image data input port 75 represent an input interface 77 of the controller 39.

The controller 39 also has an output interface 79 which is embodied as a correction data output port 81 via which correction data 83 (which is indicated by a sequence of a triangular and rectangular shaped electric signal) may be provided to the rotational stage 45, which may be referred to as movable readjustment assembly 45a.

The controller 39 further comprises a pattern recognition module 84, a stereoscopic imaging module 85, a storage module 87 in which an assignment table 89 (schematically shown) is stored and a calculation module 91.

The position recognition module 64 may be configured to identify at least one pattern, such as a blood vessel, in the stereoscopic image data 69, and to track the pattern in the images obtained at the various positions 13a-c.

The position sensor 41 may comprise the pattern recognition module 84, i.e. in this embodiment the generation of position data 65 by the position sensor 41 is based, at least partly, on pattern recognition.

The generation of correction data 83 may be based on position data 65 provided by the position sensor 41, wherein (a) the controller 39 reads assignment data 90 from the assignment table 89 from the storage module 87 and correlates the position data 65 with a necessary correction data 83 or (b) the controller 39 provides the position data 65 to the calculation module 91 which subsequently calculates the correction data 83.

It is also possible that the stereoscopic image data 69 provided by the stereoscopic cameras 43 may be applied to correlate (via the assignment table 89) or calculate (via the calculation module 91) the correction data 83 which is provided to the movable readjustment assembly 45a.

Furthermore, the correction data 83 may also be retrieved from image data 73 provided by the image sensor 47, wherein the pattern recognition module 84 of the controller 39 is adapted to identify a preferentially three-dimensional pattern 93 of a structure 94 at or in the object 33 and calculates position data 65 from the pattern 93. For example, a pattern 93 that has been identified, manually or automatically, in the initial position 13a, will have different geometry from the other positions 13b, 13c. The amount and shape of distortion of the pattern 93 allows computing the tilt of the viewing axis due to the position change. Position information from the optical assembly such as distance setting and/or focal length allow determining the position of the optical assembly relative to the identified pattern. This allows adjusting the optical assembly without the need of sensors acquiring position data directly from housing elements such as the housing.

For tilting the optical assembly, a simple control loop may be implemented which drives the tilt of the optical assembly to counteract any relative movement of the at least one identified pattern in subsequent image data. Thus, the identical pattern 93 is simply kept at a constant location within the field of view. Alternatively or additionally, the tilting may be computed by triangulation of the at least one identified pattern.

The beam path correction assembly 61 is adapted to correct the beam path 59 such that even after a rotation of the optical assembly 7 (see Fig. 1) the optical path 59 is correctly focused on the image sensor 47 and into the optical observation means 57.

A change of the working distance 25 may move the object 33 out of focus of the optical assembly 7, wherein this misalignment may be compensated by the tunable lens 53, which is configured to alter an effective focal length (not shown) of the optical assembly 7.

The medical observation apparatus 1 may be controlled by a computer 97 which reads a non-transient computer readable storage medium 95 which comprises a program for executing the inventive method.

### REFERENCE NUMERALS

- 1: medical observation apparatus
- 2: microscope
- 5: housing
- 7: optical assembly
- 9: objective
- 11: movement
- 13a: first position
- 13b: second position
- 13c: third position
- 15: initial position
- 17: viewing axis
- 19: center point
- 21: virtual sphere
- 23: radius
- 25: working distance
- 25b: second working distance
- 25c: third working distance
- 27a: first tilt angle
- 27b: second tilt angle
- 27c: third tilt angle
- 29: lens adjustment assembly
- 31: field of view
- 31a: first field of view
- 31b: second field of view
- 31c: third field of view
- 33: object
- 35a: first reference point
- 35b: second reference point
- 35c: third reference point
- 39: controller
- 41: position sensor
- 42: position data interface
- 43: stereoscopic camera
- 45: rotational stage
- 45a: moveable readjustment assembly
- 49: optical system
- 51: lens
- 53: tunable lens
- 55: beam splitter
- 57: optical observation means
- 59: optical path
- 61: beam path correction assembly
- 63: center of rotation
- 65: position data
- 67: position data input port
- 69: stereoscopic image data
- 71: stereoscopic image data port
- 73: image data
- 75: image data input port
- 77: input interface
- 79: output interface
- 81: correction data output port
- 83: correction data
- 84: parallel recognition molecule
- 85: stereoscopic imaging module
- 87: storage module
- 89: assignment table
- 90: assignment data
- 91: calculation module
- 93: pattern
- 94: structure
- 95: non-transient computer readable storage medium
- 97: computer

## Claims

1. Method for observing an object (33) using a medical observation apparatus (1), such as a microscope (3), wherein an optical assembly (7) is directed to an object (33) located in a field of view (31), and wherein the object (33) is automatically kept in the field of view (31) when the optical assembly (7) is manually moved, essentially perpendicularly to a viewing axis (17) of the optical assembly (7).

2. The method according to claim 1, wherein the method comprises the steps of receiving position data (65) from a position sensor (41) and readjusting the optical assembly (7) based on the position data (65).

3. The method according to claim 1 or 2, wherein the method further comprises the steps of recognizing at least one pattern (93) of the object (33) and retrieving position data (65) based on a variation of the at least one pattern (93), wherein the position data (65) are applied for readjusting the optical assembly (7) to keep the object in focus.

4. The method according to claim 3, wherein recognizing the at least one pattern (93) comprises stereoscopic imaging of the object (33).

5. The method according to any one of claims 2 to 4, wherein the method further comprises the steps of reading-out assignment data (90) from an assignment table (89), correlating the position data (65) with the correction data (83) and readjusting the optical assembly (7) based on correlated correction data (83).

6. The method according to any one of claims 1 to 5, wherein the method further comprises computing the correction data (83) from the position data (65) and readjusting the optical assembly (7) based on the calculated correction data (83).

7. Non-transient computer readable storage medium (95) comprising a program for executing the method according to any one of claims 1 to 6.

8. Medical observation apparatus (1), such as a microscope (3), for observing an object (33), the apparatus (1) comprising an optical assembly (7) providing an optical viewing axis (17) and a field of view (31), and a housing (5) for supporting the optical assembly (7), wherein the optical assembly (7) is adapted to be moved manually essentially perpendicularly to the optical viewing axis (17) from one position (13a) to another position (13b) and wherein a lens adjustment assembly (29) is configured to be automatically directed to the object (33) in dependence on position data (65) representative of the position (13a, 13b) of the manually moved optical assembly (7).

9. Medical observation apparatus (1) according to claim 8, wherein a position sensor (41) for generating position data (65) representing the position and/or angular orientation of the optical assembly (7) is provided.

10. Medical observation apparatus (1) according to claim 9, wherein the position sensor (41) comprises a pattern recognition module (84) for identifying at least one structure (94) and its orientation relative to the optical assembly (7) in the input image data (73).

11. Medical observation apparatus (1) according to claim 10, wherein the pattern recognition module (84) comprises a stereoscopic imaging module (85).

12. Medical observation apparatus (1) according to any one of claims 8 to 11, wherein a storage module (87) is comprised for storing assignment data (90) which correlate position data (65) with correction data (83), and wherein the position of the optical assembly (7) is adjusted based on the correction data (83).

13. Medical observation apparatus (1) according to any one of claims 8 to 12, wherein a calculation module (91) is comprised for computing correction data (83) based on the position data (65), and wherein the position of the optical assembly (7) is adjusted based on the correction data (83).

14. Medical observation apparatus (1) according to any one of claims 8 to 13, wherein the lens adjustment assembly (29) comprises a controller (39) having an input interface (77) for receiving position data (65) of the optical assembly (7), further having an output interface (79) for providing correction data (83) to a movable readjustment assembly (45a) for readjusting the optical assembly (7) to keep the object in focus.
